# EUROPÄISCHE PATENTSCHRIFT

(11) **EP 3 773 359 B1**
(45) Veröffentlichungstag und Bekanntmachung des Hinweises auf die Patenterteilung: **01.10.2025**
(21) Anmeldenummer: 19716098.9
(22) Anmeldetag: 27.03.2019
(51) Int. Cl.: A61F 2/966, A61F 2/95

(54) **IMPLANTATIONSVORRICHTUNG**
IMPLANTATION DEVICE
DISPOSITIF D'IMPLANTATION

(30) Priorität: 28.03.2018 DE 202018101750 U
(43) Veröffentlichungstag der Anmeldung: 17.02.2021
(73) Patentinhaber: VesselSens GmbH, 53175 Bonn (DE)
(72) Erfinder: DOMNICH, Alexej, 53299 Bonn (DE); SALEM, Saad, 53175 Bonn (DE)
(74) Vertreter: Pfenning, Meinig & Partner mbB
(86) Internationale Anmeldenummer: PCT/EP2019/057733
(87) Internationale Veröffentlichungsnummer: WO 2019/185724

(56) Entgegenhaltungen:
- US-A1- 2006 282 150
- US-A1- 2013 158 655

## Beschreibung

Die Erfindung betrifft eine Implantationsvorrichtung zum Implantieren eines vaskulären Implantats, die eine einfache Positionierung und schlupffreie Entladung eines vaskulären Implantats ermöglicht.

US 2013/158655 A1 zeigt einen Griff zur Verwendung mit einem perkutanen Einführsystem für den Aortenklappenersatz.

Implantationsbesteck und Katheter werden zur Einführung und Positionierung von medizinischen Implantaten in Blutgefäßsystemen verwendet, zum Beispiel für den Einsatz in der Humanmedizin. In der sogenannten perkutanen transluminalen Angioplastie und der perkutanen transluminalen koronaren Angioplastie werden minimalinvasive Implantate wie zum Beispiel Stents mit Implantationsbesteck (Delivery System) an den Ort des betroffenen Gefäßabschnitts über die Gefäße vorgeschoben und appliziert. Für die Positionierung des Katheters im Gefäß wird ein wesentlich kleinerer Durchmesser das Katheters gewählt als das betroffene Gefäß mit der Engstelle, die der Katheter durchdringen muss. Bei selbstexpandierenden Implantaten wie beispielsweise Nitinol-Stents wird hierfür eine Schleuse (Schlauch) auf dem Kathetersystem verwendet. Sobald das Kathetersystem am verengten Abschnitt des Gefäßes angelangt ist, wird die Schleuse gespült und anschließend relativ zum Kathetersystem nach hinten geschoben. Dabei entfaltet sich der selbstexpandierende Stent oberhalb der Af-Temperatur (Form-Gedächtnis-Legierung), wobei die Af-Temperatur deutlich geringer als die Körpertemperatur gewählt wird.

Wichtig für die Positionierungsgenauigkeit des Implantats im Gefäß ist die Relativbewegung der Schleuse auf dem fixen Kathetersystem. Üblicherweise fixiert der Operateur zwar das System mit einer Hand und zieht mit der anderen die Schleuse zurück, des Öfteren kommt es jedoch vor, dass es zu einer geringfügigen Relativbewegung zwischen der Schleuse und dem Kathetersystem kommt, die zu einer fehlerhaften Implantation und somit zu schwerwiegenden Komplikationen führt.

Insbesondere bei Implantaten, die auch Sensorfunktionen umfassen, ist häufig aufgrund der geometrischen Eigenschaften die Reibungskraft zwischen der verschiebbaren Schleuse und dem Implantat erhöht. Dadurch muss für den Entladevorgang des Sensorsystems vom Anwender eine große Kraft aufgebracht werden, um das Implantat zu entlassen. Bei großen Reibungskräften kann es hier zu einer unerwünschten Relativbewegung des gesamten Kathetersystems bezüglich der Schleuse kommen, wodurch der Entladevorgang entweder scheitert oder fehlerhaft erfolgt.

Aus dem Stand der Technik sind Lösungen bekannt, die unter anderem auch im kardiovaskulären Bereich Einsatz finden. Solche Systeme werden beispielsweise für Stentgrafts oder für die Implantation von Herzklappenersatz verwendet.

Aufgabe der vorliegenden Erfindung ist es, eine Implantationsvorrichtung zum Implantieren eines vaskulären Implantats anzugeben, die eine sicherere Implantation erlaubt.

Die Aufgabe wird gelöst durch die Implantationsvorrichtung nach Anspruch 1. Die abhängigen Ansprüche geben vorteilhafte Weiterbildungen erfindungsgemäßen Implantationsvorrichtung an.

Erfindungsgemäß wird eine Implantationsvorrichtung zum Implantieren eines vaskulären Implantats angegeben. Die erfindungsgemäße Implantationsvorrichtung weist ein innenverzahntes Zahnrad auf, das vorteilhaft zylinderförmig ausgestaltet sein kann.

Die erfindungsgemäße Implantationsvorrichtung weist außerdem eine oder mehrere nebeneinander angeordnete Transportvorrichtungen auf. Jede der Transportvorrichtungen weist jeweils eine Spindel und ein koaxial zur Spindel angeordnetes außenverzahnten Zahnrad auf. Dabei ist das außenverzahnte Zahnrad mit der Spindel fest verbunden. Vorteilhafterweise ist eine Drehachse des außenverzahnten Zahnrads koaxial zu einer Achse, um welche die Spindel gewunden ist. Vorteilhafterweise haben die Spindeln aller Transportvorrichtungen die gleiche Helizität.

Besonders vorteilhaft kann die Spindel und das außenverzahnten Zahnrad der Transportvorrichtungen jeweils durch eine gemeinsame Welle getragen werden, die koaxial liegt zur Drehachse des außenverzahnten Zahnrades.

Die erfindungsgemäße Implantationsvorrichtung weist außerdem zumindest einen Adapter auf, der eine der Anzahl der Transportvorrichtungen entsprechende Anzahl an Gewinden aufweist. Die Gewinde sind dabei vorteilhafterweise schraubenförmige Innengewinde. Die Gewindeachsen der Gewinde liegen vorteilhafterweise parallel zueinander.

In der erfindungsgemäßen Implantationsvorrichtung sind die Zahnräder der einen oder mehreren Transportvorrichtungen mit dem innenverzahnten Zahnrades im Eingriff. Auf diese Weise wird eine Drehung des innenverzahnten Zahnrades auf die außenverzahnten Zahnräder der Transportvorrichtungen übertragen, die wiederum eine Drehung der Spindeln bewirken.

Erfindungsgemäß sind die Spindeln der einen oder mehreren Transportvorrichtungen jeweils mit einem der Gewinde des Adapters im Eingriff. Vorteilhafterweise haben also die Innengewinde des Adapters jeweils die gleiche Steigung wie die Spindel, die in dieses Gewinde eingreift. Eine Drehung der Spindeln führt daher zu einer Bewegung des Adapters in Richtung der Achse der Spindeln.

Die erfindungsgemäße Implantationsvorrichtung weist außerdem eine rohrförmige Schleuse auf, die an einem ihrer Enden mit dem Adapter verbunden ist. Eine Bewegung des Adapters führt also zu einer Bewegung der Schleuse.

Darüberhinaus weist die Implantationsvorrichtung ein Kanülenrohr auf, das über zumindest die Länge der Spindeln neben den Spindeln der einen oder mehreren Transportvorrichtungen und parallel zu diesen angeordnet ist.

Die Implantationsvorrichtung weist außerdem einen Katheter auf, der zumindest teilweise im Kanülenrohr angeordnet ist. Vorzugsweise ist der Katheter mit dem Kanülenrohr an dessen einen Ende verklebt.

In einer vorteilhaften Ausgestaltung der Erfindung können das innenverzahnte Zahnrad, die eine oder mehrere Transportvorrichtungen und der Adapter in einem gemeinsamen Gehäuse angeordnet sein. Das Kanülenrohr kann dann an einem distalen Ende des Gehäuses gehalten werden und zumindest bis zu einem gegenüberliegenden proximalen Ende des Gehäuses verlaufen. Unter dem proximalen Ende wird dabei jenes Ende verstanden, das bei bestimmungsgemäßer Verwendung der Implantationsvorrichtung einem Patienten zugewandt ist. Entsprechend ist das distale Ende jenes Ende, das bei bestimmungsgemäßer Verwendung vom Patienten weggewandt ist. Vorteilhafterweise ist der Katheter mit jenem Ende des Kanülenrohres verbunden, an dem das Kanülenrohr am Gehäuse gehalten wird.

In einer vorteilhaften Ausgestaltung der Erfindung kann das Kanülenrohr an jenem Ende, an dem es am Gehäuse gehalten wird, in ein Flügelelement eingesteckt sein. Dabei kann das Flügelelement eine zylinderförmige Ausnehmung aufweisen, in die das Kanülenrohr eingesteckt ist. An einem Außenumfang der zylinderförmigen Ausnehmung können zwei einander bezüglich einer Zylinderachse gegenüberliegende Flügelflächen angeordnet sein. Vorteilhafterweise kann das Flügelelement in Richtung der Zylinderachse sich über die Flügelelemente hinaus erstreckende Abschnitte aufweisen, die einen kreisförmigen Umfang haben. In diesen Abschnitten kann das Flügelelement in dem Gehäuse gelagert sein.

In einer vorteilhaften Ausgestaltung der Findung kann die Innenverzahnung des innenverzahnten Zahnrades 40 Zähne aufweisen und jeweils das Zahnrad der einen oder mehreren Transportvorrichtungen 10 Zähne aufweisen. Eine solche Bemessung ermöglicht eine gute Kraftübertragung von dem innenverzahnten Zahnrad auf das oder die außenverzahnten Zahnräder und damit auf die Spindeln.

In einer besonders vorteilhaften Ausgestaltung der Erfindung kann das Gehäuse zumindest zwei Öffnungen aufweisen, durch welche das innenverzahnte Zahnrad jeweils hindurchragt. Auf diese Weise kann ein Verwender der Vorrichtung das innenverzahnte Zahnrad greifen und drehen. Die Öffnungsflächen dieser Öffnungen können dabei vorteilhaft zylinderförmig sein und parallel liegen zu einer Außenoberfläche des innenverzahnten Zahnrades. Insbesondere ist es auch möglich, dass die Öffnungsflächen der Öffnungen zusammenfallen mit der Außenoberfläche des innenverzahnten Zahnrades im Bereich der jeweiligen Öffnung. Vorteilhafterweise können die zumindest zwei Öffnungen einander bezüglich einer Drehachse des innenverzahnten Zahnrades genau gegenüber liegen.

In einer vorteilhaften Ausgestaltung der Erfindung kann die Implantationsvorrichtung ein Gegenzahnrad aufweisen, das im Inneren des innenverzahnten Zahnrades angeordnet ist und mit den Verzahnungen der Zahnräder der Transportvorrichtungen im Eingriff ist.

Das Gegenzahnrad kann hierbei ein außenverzahnten Zahnrad sein. Die Bemessung des Gegenzahnrades ergibt sich aus den Abmessungen des innenverzahnten Zahnrades und der Zahnräder der Transportvorrichtungen. Vorteilhafterweise kann das Gegenzahnrad koaxial zu einer Drehachse des innenverzahnten Zahnrades angeordnet sein.

Bevorzugterweise weist die erfindungsgemäße Implantationsvorrichtung genau zwei der Transportvorrichtungen auf. Besonders bevorzugt sind die zwei Transportvorrichtungen dann bezüglich des Kanülenrohres gegenüber angeordnet. Vorteilhafterweise liegen die Achsen der außenverzahnten Zahnräder und die Spindeln dieser Transportvorrichtungen parallel zueinander.

In einer vorteilhaften Ausgestaltung der Erfindung können die Spindeln jeweils eine Länge von größer oder gleich 80 mm, vorzugsweise größer gleich 90 mm, besonders bevorzugt größer gleich 100 mm und/oder kleiner oder gleich 130 mm, vorzugsweise kleiner oder gleich 120 mm haben. Die Länge der Spindeln wird vorzugsweise abhängig von der Länge des zu implantieren Implantats festgelegt und ist vorzugsweise zumindest so lang, wie die Länge des Implantats.

Eine Steigung der Spindeln kann vorzugsweise größer oder gleich 4 mm, vorzugsweise größer gleich 5 mm, vorzugsweise gleich 6 mm und/oder kleiner oder gleich 9 mm, vorzugsweise kleiner oder gleich 7 mm betragen.

Vorteilhafterweise kann die Spindel als Trapezgewinde ausgestaltet sein.

In einer vorteilhaften Ausgestaltung der Findung kann die rohrförmige Schleuse mit dem Adapter über eine Schraubkappe und/oder einen Ring mit Fase verbunden sein.

In einer vorteilhaften Ausgestaltung der Erfindung kann das Gehäuse auf seiner Außenseite eine lineare Skala aufweisen. Es kann dann der Adapter ein Element aufweisen, das durch einen parallel zur Skala neben der Skala verlaufenden Einschnitt im Gehäuse hindurchragt. Bevorzugterweise ist dieser Einschnitt als länglicher Schlitz ausgebildet, der parallel zum Kanülenrohr liegt. Das Element, das aus dem Gehäuse durch den Einschnitt hindurchragt, dient dann als Anzeiger der Position des Adapters auf der Skala. Auf diese Weise kann die Position des Adapters an der Skala abgelesen werden und damit abgelesen werden, wie weit das Implantat bereits freigesetzt wurde.

Das innenverzahnte Zahnrad, die eine oder mehreren Transportvorrichtungen, der Adapter und das Kanülenrohr, wie auch gegebenenfalls das Gegenzahnrad sind vorteilhafter Weise im Inneren des Gehäuses angeordnet. Das Gehäuse kann einen proximalen Abschnitt aufweisen, in dem die Spindeln und das Kanülenrohr vorliegen, und in dem der Adapter verschiebbar ist. Auf seiner distalen Seite kann dieser Abschnitt des Gehäuses durch eine Wand begrenzt werden, durch die hindurch die Wellen der Transportvorrichtungen verlaufen, so dass die Spindeln auf der proximalen Seite dieser Wand angeordnet sind und die außenverzahnten Zahnräder der Transportvorrichtungen auf der distalen Seite dieser Wand. Auf der distalen Seite der Wand kann sich dann ein Bereich des Gehäuses anschließen, in dem das innenverzahnte Zahnrad angeordnet ist. Dieser Bereich des Gehäuses kann die beschriebenen Öffnungen aufweisen, durch die hindurch das innenverzahnte Zahnrad von einem Benutzer drehbar ist.

Auf der distalen Seite des innenverzahnten Zahnrades kann sich dann noch ein Bereich des Gehäuses anschließen, in dem das Kanülenrohr und der Katheter gehalten werden. Dieser Bereich kann gegenüber den anderen proximalen Bereichen des Gehäuses verjüngt sein.

Im folgenden soll die Erfindung mit Bezugnahme auf einige Figuren beispielhaft erläutert werden. Gleiche Bezugszeichen kennzeichnen dabei gleiche oder entsprechende Merkmale. Die in den Beispielen beschriebenen Merkmale können auch unabhängig vom konkreten Beispiel realisiert werden und zwischen verschiedenen Beispielen kombiniert werden.

Es zeigt
Figur 1
   eine Explosionszeichnung einer erfindungsgemäßen Implantationsvorrichtung,
Figur 2
   einen Schnitt durch einen innenverzahntes Zahnrad mit außenverzahnten Zahnrädern und einem Gegenzahnrad, und
Figuren 3 und 4
   ein Beispiel eines Sensorimplantats, das mit der erfindungsgemäßen Implantationsvorrichtung implantierbar ist.

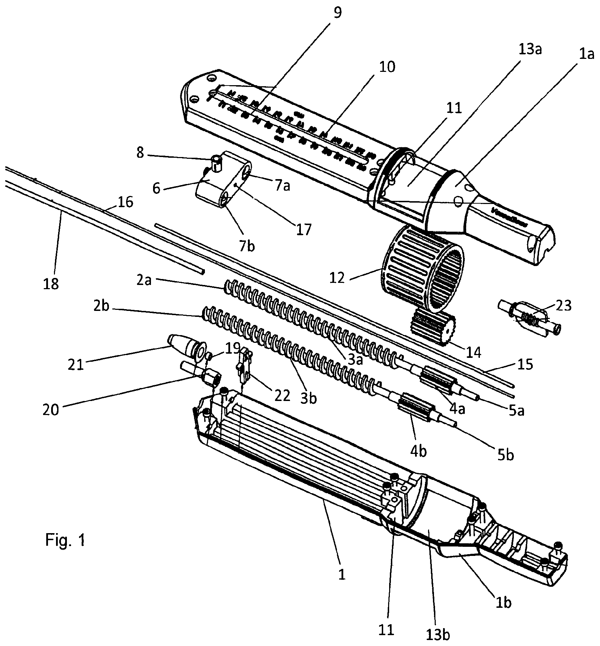

Figur 1 zeigt ein Beispiel einer erfindungsgemäßen Implantationsvorrichtung als Explosionszeichnung. Im gezeigten Beispiel weist die Implantationsvorrichtung ein Gehäuse 1 auf, dass hier zweiteilig mit einer Oberschale 1a und einer Unterschale 1b ausgestaltet ist. Im folgenden soll die linke Seite des Gehäuses als proximale Seite und die rechte Seite als distale Seite bezeichnet werden. Im Inneren des Gehäuses sind zwei Transportvorrichtungen 2a und 2b angeordnet, die jeweils eine Spindel 3a, 3b sowie ein außenverzahntes Zahnrad 4a, 4b aufweisen. Die Spindeln 3a, 3b und die außenverzahnten Zahnräder 4a, 4b der Transportvorrichtungen 2a und 2b werden jeweils durch eine gemeinsame Welle 5a bzw. 5b getragen.

Im Inneren des Gehäuses 1 ist außerdem einen Adapter 6 angeordnet, der zwei Innengewinde 7a und 7b aufweist, in welche die Spindeln 3a und 3b der Transportrichtungen eingreifen. Die Spindeln 3a und 3b können im proximalen Bereich des Gehäuses fest gelagert sein und im distalen Bereich lose gelagert.

Der Adapter 6 weist ein Element 8 auf, das durch einen Einschnitt 9 in der Oberschale 1a des Gehäuses hindurchragt, sodass es von außen auf der Oberschale 1a sichtbar ist. Die Oberschale 1a weist entlang des Einschnitts 9 eine Skala 10 auf. Verschiebt sich der Adapter 6 im Inneren des Gehäuses, so verschiebt sich das Element 8 entlang der Skala 10, sodass die Position des Adapters 6 an der Skala 10 ablesbar ist.

Das Gehäuse weist in seinem Inneren eine Wand 11 auf, die einen proximalen Abschnitt des Gehäuses zur distalen Seite hin begrenzt. Die Wellen 5a und 5b der Transportvorrichtungen 2a, 2b treten durch die Wand 11 hindurch. Die außenverzahnten Zahnräder 4a, 4b der Transportvorrichtungen 2a, 2b liegen dann auf der distalen Seite der Wand 11.

Die Spindeln 3a, 3b sind im proximalen Bereich des Gehäuses angeordnet. Durch Drehen der Spindeln 3a, 3b ist der Adapter 6 in diesem Bereich des Gehäuses zwischen dem proximalen Ende des Gehäuses und der Wand 11 verschiebbar.

Ein innenverzahntes Zahnrad 12 ist in einem Bereich des Gehäuses distal der Wand 11 angeordnet. In diesem Bereich weist das Gehäuse zwei Öffnungen 13a und 13b auf, durch die das innenverzahnte Zahnrad 12 hindurchragt. Durch die Öffnungen 13a und 13b ist das innenverzahnte Zahnrad 12 von außen drehbar.

Die außenverzahnten Zahnräder 4a und 4b der Transportvorrichtungen 2a und 2b sind mit der Innenverzahnung des innenverzahnten Zahnrades 12 im Eingriff. Im Inneren des innenverzahnten Zahnrades 12 ist außerdem ein Gegenzahnrad 14 angeordnet, dass mit beiden außenverzahnten Zahnrädern 4a, 4b der Transportvorrichtungen 2a, 2b im Eingriff ist. Das Gegenzahnrad 14 liegt dabei koaxial zu einer Zylinderachse des innenverzahnten Zahnrades 12. Im Inneren des Gehäuses verläuft ein Kanülenrohr 15, dass so angeordnet ist, dass es genau in der Mitte zwischen den Transportvorrichtungen 2a und 2b liegt. Der Adapter 6 weist mittig zwischen den Innengewinden 7a, 7b eine Durchgangsöffnung 17 auf, durch die das Kanülenrohr 15 hindurch verläuft, sodass der Adapter 6 entlang des Kanülenrohres 15 verschiebbar ist.

Die in Figur 1 gezeigte Vorrichtung weist einen Innenkatheter 16 auf, der teilweise im Inneren des Kanülenrohrs 15 verläuft. Der Katheter 16 ist mit dem Kanülenrohr 15 an dessen distalen Ende verklebt.

Der Adapter 6 ist auf seiner proximalen Seite mit einer Schleuse 18 verbunden. Die Verbindung zwischen der Schleuse 18 und dem Adapter 6 kann beispielsweise über eine Schraubkappe 20 und einen Edelstahlring mit Fase 19 hergestellt sein. Um die Schraubkappe 20 und den Edelstahlring 19 kann eine Tülle 21 als Biege,- und Knickschutz sowie zur exakten Führung der Schleuse 18 beim Abziehen vorgesehen sein.

Zur Dreh- und Transportsicherung des Gehäuses kann ein Stopper 22 vorgesehen sein, der beispielsweise durch den Einschnitt 9 eingesetzt werden kann und im eingesetzten Zustand die Bewegung des Adapters 6 blockiert. Der Stopper 22 kann hierzu beispielsweise Zapfen aufweisen, die in entsprechende Öffnungen auf der Oberseite der Oberschale 1a eingreifen.

Das Kanülenrohr 15 kann an seinem distalen Ende in ein Flügelbauteil 23 eingesteckt sein, das in einem das Gehäuse in distale Richtung begrenzenden Bereich liegt.

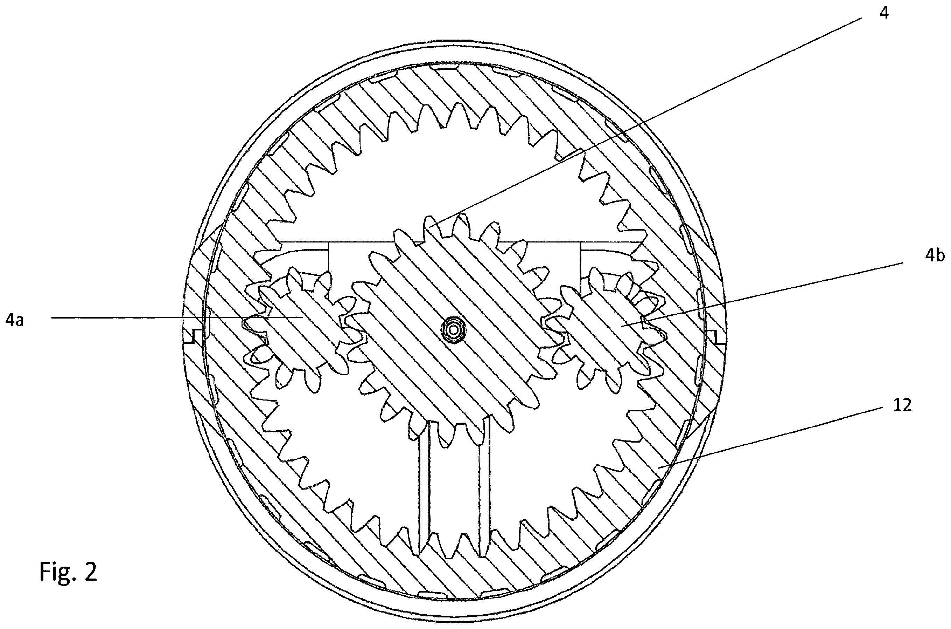

Figur 2 zeigt einen Schnitt durch die erfindungsgemäße Implantationsvorrichtung gemäß Figur 1 im Bereich des innenverzahnten Zahnrades 12. Die Schnittebene liegt dabei senkrecht zu den Längsrichtungen der Wellen 5a, 5b der Transportvorrichtungen 2a, 2b.

Wird das innenverzahnte Zahnrad 12 durch einen Benutzer wie gezeigt im Uhrzeigersinn gedreht, so drehen sich die außenverzahnten Zahnräder 4a, 4b der Transportvorrichtungen 2a, 2b ebenfalls im Uhrzeigersinn. Das zentral angeordnete Gegenzahnrad 4 ist mit den Zahnrädern 4a, 4b im Eingriff und dreht sich in diesem Fall entgegen dem Uhrzeigersinn. Durch das Gegenzahnrad 14 kann das Drehmoment zwischen den Zahnrädern 4a, 4b übertragen werden.

Im gezeigten Beispiel hat das innenverzahnte Zahnrad 12 40 Zähne und die beiden außenverzahnten Zahnräder 4a, 4b jeweils 10 Zähne. Die Übersetzung des Zahnradgetriebes beträgt also im gezeigten Beispiel 4 und alle Zähne haben ein Eingriffswinkel von 20°.

Beträgt die Länge des Implantats 100 mm, so ist es vorteilhaft, wenn der durch die Schleuse 18 zurückgelegte Weg bei der Implantation mindestens 100 mm beträgt. Im gezeigten Beispiel wurde für das Zurücklegen dieses Weges die Steigung der Gewindeflanken der Spindeln 3a, 3b auf 6 mm festgelegt. In Kombination mit der gewählten Übersetzung des Zahnradgetriebes ist also eine komplette Entlassung des Implantats mit 4,16 Umdrehungen des innenverzahnten Zahnrades 12 möglich. Das Gewinde der Spindel kann als Trapezgewinde ausgeführt sein, was besonders gut zur Übertragung der Bewegungen und Kräfte geeignet ist.

Die Skala 10 auf der sichtbaren Oberfläche der Oberschale 1a in Zentimetern dient zum Ablesen des zurückgelegten Verfahrenswegs.

Die Implantationsvorrichtung der Erfindung kann beispielsweise zu Entlassung von peripheren Stents, zu Entlassung von mehreren peripheren Stents mit definierten Abständen und zu Entlassung von Stentgrafts verwendet werden. Insbesondere kann sie zur Entlassung von vaskulären und kardiovaskulären Implantaten verwendet werden.

Die erfindungsgemäße Implantationsvorrichtung ermöglicht eine einfache Positionierung und schlupffreie Entladung eines Implantats mit höherer Kraft und Momentübertragung, als es mit Systemen des Standes der Technik möglich ist. Durch die Verringerung des Kraftaufwands wird das Risiko einer Relativbewegung des Katheters zur Schleuse während des Implantationsvorgangs reduziert.

Eine Spülung des Kathetersystems kann durch einen Luer-Lock-Anschluss im Adapter 6 durchgeführt werden. Ein Führungsdraht hingegen kann durch das Flügelelement 23 eingeschoben werden.

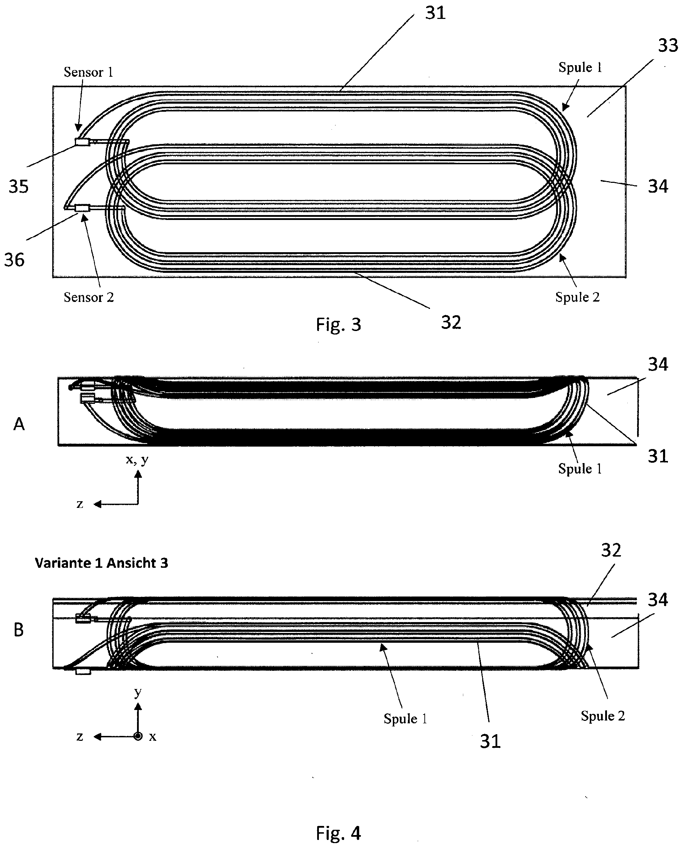

Figur 3 zeigt ein Beispiel eines Sensorimplantats, das mit der erfindungsgemäßen Implantationsvorrichtung implantierbar ist. Das Sensorimplantat umfasst eine gedruckte Schaltung 33 mit einer ersten Leiterschleifenstruktur 31 und einer zweiten Leiterschleifenstruktur 32. Dabei ist die Leiterschleifenstruktur 31 auf einer Vorderseite, in der Figur 3 dem Betrachter zugewandt, eines Substrates 34 aufgebracht und die zweite Leiterschleifenstruktur 32 auf einer dem Betrachter abgewandten Rückseite des Substrats 34. Das Substrat 34 isoliert also die erste Leiterschleifenstruktur 31 und die zweite Leiterschleifenstruktur 32 elektrisch gegeneinander.

Die Leiterschleifenstrukturen 31 und 32 haben im gezeigten Beispiel jeweils drei Windungen. Jede der Leiterschleifenstrukturen 31 und 32 weist zwei gerade Bereiche auf, die zueinander parallel liegen und über zwei kreisförmig gebogene Bereiche miteinander verbunden sind. In den geraden Bereichen verlaufen die Leiterbahnen parallel zueinander und gerade und in den kreisförmig gebogenen Bereichen verlaufen die Leiterbahnen entlang einer Kreislinie und für alle Windungen der gleichen Leiterschleifenstruktur parallel zueinander. Mit der ersten Leiterschleifenstruktur 31 ist ein erster kapazitiver Drucksensor 35 gekoppelt. Der kapazitive Drucksensor 35 ist dabei zwischen den zwei Enden der Leiterschleifenstruktur 31 gekoppelt. Entsprechend weist die Leiterschleifenstruktur 32 einen zweiten kapazitiven Drucksensor 36 auf, der wiederum zwischen den beiden Enden der Leiterschleifenstruktur 32 angeordnet ist. Der erste kapazitive Drucksensor 35 bildet mit der ersten Leiterschleifenstruktur 31 einen ersten Schwingkreis mit einer ersten Resonanzfrequenz. Der zweite kapazitive Drucksensor 36 bildet mit der zweiten Leiterschleifenstruktur 32 einen zweiten Schwingkreis mit einer zweiten Resonanzfrequenz.

Die erste Leiterschleifenstruktur 31 ist um eine Windungsachse gewunden, wobei die Windungsachse hier den Mittelpunkt der Leiterbahnen der Leiterschleifenstruktur 31 durchstößt und senkrecht auf dem Substrat 34 steht. In entsprechender Weise werden die Leiterbahnen der zweiten Leiterschleifenstruktur 32 um eine zweite Spulenachse gewunden, die wiederum durch den Mittelpunkt der Leiterbahn der zweiten Leiterschleifenstruktur 32 verläuft und senkrecht auf dem Substrat 34 steht.

Aus der in Figur 3 gezeigten Struktur kann ein wie in Figur 4 gezeigtes Implantat hergestellt werden, indem das Substrat 34 um eine Achse gebogen wird, die parallel zu den Längsseiten des Substrats 34 verläuft, die parallel liegen zu den geraden Abschnitten der Leiterschleifenstrukturen 31 und 32. Diese Richtung soll im Folgenden als Z-Richtung bezeichnet werden.

Figur 4A zeigt diese Ausgestaltung des Transponders in einer ersten Richtung, die senkrecht zur Z-Richtung und in einem Winkel von 45° zur X-Achse und zur Y-Achse steht. Die Teilfigur 4B zeigt den Transponder in Richtung der X-Achse betrachtet.

Die Leiterschleifenstrukturen 31 und 32 können vorteilhaft so bemessen sein, dass, wenn das Substrat 34 in der beschriebenen Weise gebogen wird, die geraden Bereiche der Leiterschleifenstrukturen einander gerade diametral bezüglich jener Achse, um die das Substrat 34 gebogen wurde, gegenüberliegen. Das Substrat 34 wird dabei vorzugsweise in eine Kreisform so gebogen, dass die Spulenachsen der ersten Leiterschleifenstruktur und der zweiten Leiterschleifenstruktur 32 im gewünschten Winkel zueinander stehen, vorzugsweise senkrecht zueinander. Die Erfindung gemäß Implantationsvorrichtung eignet sich zur Implantation eines solchen Implantats.

## Patentansprüche

1. Implantationsvorrichtung zum Implantieren eines vaskulären Implantats, aufweisend
ein innenverzahntes Zahnrad (12),
eine oder mehrere nebeneinander angeordnete Transportvorrichtungen (2a, 2b) mit jeweils einer Spindel (3a, 3b) und einem koaxial zur Spindel (3a, 3b) angeordneten außenverzahnten Zahnrad (4a, 4b), das mit der Spindel (3a, 3b) fest verbunden ist,
zumindest einen Adapter (6) mit einer der Anzahl der Transportvorrichtungen entsprechenden Anzahl an Gewinden (7a, 7b),
wobei die Zahnräder (4a, 4b) der einen oder mehreren Transportvorrichtungen (2a, 2b) mit dem Innengewinde des innenverzahnten Zahnrades (12) im Eingriff sind,
und wobei die Spindeln (3a, 3b) der einen oder mehreren Transportvorrichtungen (2a, 2b) jeweils mit einem der Gewinde (7a, 7b) des Adapters (6) im Eingriff sind,
weiter aufweisend
eine rohrförmige Schleuse (18) , die an einem ihrer Enden mit dem Adapter (6) verbunden ist,
ein Kanülenrohr (15), das über zumindest die Länge der Spindeln (3a, 3b) neben den Spindeln (3a, 3b) der einen oder mehreren Transportvorrichtungen (2a, 2b) und parallel zu diesen angeordnet ist,
und ein Katheter (16), der zumindest teilweise innerhalb des Kanülenrohrs (15) angeordnet ist.

2. Implantationsvorrichtung nach dem vorhergehenden Anspruch, wobei das innenverzahnte Zahnrad (12), die eine oder mehrere Transportvorrichtungen (2a, 2b) und der Adapter (6) in einem gemeinsamen Gehäuse (1) angeordnet sind,
wobei das Kanülenrohr (15) an einem distalen Ende des Gehäuses (1) gehalten wird und zumindest bis zu einem gegenüberliegenden proximalen Ende des Gehäuses (1) verläuft und
wobei der Katheter (16) mit jenem Ende des Kanülenrohres (15) an dem das Kanülenrohr (15) am Gehäuse (1) gehalten wird, verbunden ist.

3. Implantationsvorrichtung nach einem der vorhergehenden Ansprüche, wobei das Kanülenrohr (15) an jenem Ende, an dem es am Gehäuse (1) gehalten wird, in ein Flügelelement (23) eingesteckt ist.

4. Implantationsvorrichtung nach einem der vorhergehenden Ansprüche, wobei die Innenverzahnung des innenverzahnten Zahnrades (12) 40 Zähne hat, und wobei das Zahnrad (4a, 4b) der einen oder mehreren Transportvorrichtungen (2a, 2b) jeweils 10 Zähne hat.

5. Implantationsvorrichtung nach einem der Ansprüche 2 bis 4,
wobei das Gehäuse (1) zumindest zwei Öffnungen aufweist, durch welche das innenverzahnte Zahnrad (12) jeweils hindurchragt und drehbar ist.

6. Implantationsvorrichtung nach einem der vorhergehenden Ansprüche, weiter aufweisend ein Gegenzahnrad (4) , das im Inneren des innenverzahnten Zahnrades (12) angeordnet ist und mit den Verzahnungen der Zahnräder (4a, 4b) der Transportvorrichtungen (2a, 2b) im Eingriff ist, wobei vorzugsweise das Gegenzahnrad (4) koaxial zu einer Drehachse des innenverzahnten Zahnrades (12) angeordnet ist.

7. Implantationsvorrichtung nach einem der vorhergehenden Ansprüche, aufweisend genau zwei der Transportvorrichtungen (2a, 2b), die bezüglich des Kanülenrohres (15) gegenüber angeordnet sind.

8. Implantationsvorrichtung nach einem der vorhergehenden Ansprüche, wobei eine Länge der Spindeln (3a, 3b) jeweils gleich oder größer als 100 mm beträgt.

9. Implantationsvorrichtung nach einem der vorhergehenden Ansprüche, wobei eine Steigung der Spindeln (3a, 3b) 6 mm beträgt.

10. Implantationsvorrichtung nach einem der vorhergehenden Ansprüche, wobei die Spindel (3a, 3b) als Trapezgewinde ausgestaltet ist.

11. Implantationsvorrichtung nach einem der Ansprüche 2 bis 11,
wobei das Gehäuse (1) auf einer Außenseite eine lineare Skala (10) aufweist, und wobei der Adapter (6) ein Element (8) aufweist, das durch einen parallel zur Skala (10) neben der Skala verlaufenden Einschnitt (9) im Gehäuse (1) hindurchragt.

## Claims

1. Implantation device for implanting a vascular implant, comprising
an internally toothed gear (12),
one or more transport devices (2a, 2b) arranged adjacent to one another, each with a spindle (3a, 3b) and an externally toothed gear (4a, 4b) arranged coaxially to the spindle (3a, 3b), which is firmly connected to the spindle (3a, 3b),
at least one adapter (6) with a number of threads (7a, 7b) corresponding to the number of transport devices,
wherein the gears (4a, 4b) of the one or more transport devices (2a, 2b) are in engagement with the internal thread of the internally toothed gear (12), and
wherein the spindles (3a, 3b) of the one or more transport devices (2a, 2b) are each in engagement with one of the threads (7a, 7b) of the adapter (6),
further comprising
a tubular sheath (18) which is connected at one of its ends to the adapter (6),
a hypotube (15) which is arranged over at least the length of the spindles (3a, 3b), adjacent to the spindles (3a, 3b) of the one or more transport devices (2a, 2b) and parallel to them, and
a catheter (16) which is at least partially arranged within the hypotube (15).

2. Implantation device according to the preceding claim,
wherein the internally toothed gear (12), the one or more transport devices (2a, 2b) and the adapter (6) are arranged in a common housing (1),
wherein the hypotube (15) is held at a distal end of the housing (1) and extends at least to an opposite proximal end of the housing (1) and
wherein the catheter (16) is connected to the end of the hypotube (15) at which the hypotube is (15) held on the housing (1).

3. Implantation device according to any of the preceding claims, wherein the hypotube (15) is inserted into a wing element (23) at the end at which it is held on the housing (1).

4. Implantation device according to any of the preceding claims, wherein the internal toothing of the internally toothed gear (12) has 40 teeth, and wherein the gear(s) (4a, 4b) of the one or more transport devices (2a, 2b) each have 10 teeth.

5. Implantation device according to any of claims 2 to 4, wherein the housing (1) has at least two openings through which the internally toothed gear (12) protrudes and can be rotated.

6. Implantation device according to any of the preceding claims, further comprising a counter gear (4) which is arranged inside the internally toothed gear (12) and in engagement with the teeth of the gears (4a, 4b) of the transport devices (2a, 2b), wherein the counter gear (4) is preferably arranged coaxially to an axis of rotation of the internally toothed gear (12).

7. Implantation device according to any of the preceding claims, comprising exactly two transport devices (2a, 2b) which are arranged opposite one another with respect to the hypotube (15).

8. Implantation device according to any of the preceding claims, wherein the length of the spindles (3a, 3b) is in each case equal to or greater than 100 mm.

9. Implantation device according to any of the preceding claims, wherein the pitch of the spindles (3, 3b) is 6 mm.

10. Implantation device according to any of the preceding claims, wherein the spindle (3a, 3b) is designed as a trapezoidal thread.

11. Implantation device according to any of claims 2 to 11, wherein the housing (1) has a linear scale (10) on an outer face, and wherein the adapter (6) has an element (8) which protrudes through an incision (9) in the housing (1), which extends parallel to the scale (10), adjacent to the scale.

## Revendications

1. Dispositif d'implantation pour implanter un implant vasculaire, comprenant
une roue dentée à denture intérieure (12),
un ou plusieurs dispositifs de transport (2a, 2b) disposés les uns à côté des autres, comprenant chacun une broche (3a, 3b) et une roue dentée à denture extérieure (4a, 4b) disposée coaxialement à la broche (3a, 3b) et solidement reliée à la broche (3a, 3b),
au moins un adaptateur (6) comportant un nombre de filetages (7a, 7b) correspondant au nombre de dispositifs de transport,
les roues dentées (4a, 4b) des un ou plusieurs dispositifs de transport (2a, 2b) étant en prise avec le filetage intérieur de la roue dentée à denture intérieure (12), et
les broches (3a, 3b) des un ou plusieurs dispositifs de transport (2a, 2b) étant chacune en prise avec l'un des filetages (7a, 7b) de l'adaptateur (6),
présentant en outre
un sas tubulaire (18) relié à l'une de ses extrémités à l'adaptateur (6),
un tube de canule (15) disposé sur au moins la longueur des broches (3a, 3b) à côté des broches (3a, 3b) des un ou plusieurs dispositifs de transport (2a, 2b) et parallèlement à celles-ci, et
un cathéter (16) disposé au moins partiellement à l'intérieur du tube de canule (15).

2. Dispositif d'implantation selon la revendication précédente,
dans lequel la roue dentée à denture intérieure (12), les un ou plusieurs dispositifs de transport (2a, 2b) et l'adaptateur (6) sont disposés dans un boîtier commun (1),
dans lequel le tube de canule (15) est maintenu à une extrémité distale du boîtier (1) et s'étend au moins jusqu'à une extrémité proximale opposée du boîtier (1) et
dans lequel le cathéter (16) est relié à l'extrémité du tube de canule (15) au niveau de laquelle le tube de canule (15) est maintenu sur le boîtier (1).

3. Dispositif d'implantation selon l'une quelconque des revendications précédentes, dans lequel le tube de canule (15) est inséré dans un élément d'aile (23) à l'extrémité par laquelle il est maintenu sur le boîtier (1).

4. Dispositif d'implantation selon l'une quelconque des revendications précédentes, dans lequel la denture intérieure de la roue dentée à denture interne (12) possède 40 dents, et dans lequel la roue dentée (4a, 4b) de chacun des un ou plusieurs dispositifs de transport (2a, 2b) possède 10 dents.

5. Dispositif d'implantation selon l'une des revendications 2 à 4, dans lequel le boîtier (1) présente au moins deux ouvertures à travers lesquelles la roue dentée (12) à denture intérieure fait respectivement saillie et peut tourner.

6. Dispositif d'implantation selon l'une des revendications précédentes, comprenant en outre une contre-roue dentée (4) disposée à l'intérieur de la roue dentée à denture intérieure (12) et qui est en prise avec les dentures des roues dentées (4a, 4b) des dispositifs de transport (2a, 2b), la contre-roue dentée (4) étant de préférence disposée coaxialement à un axe de rotation de la roue dentée à denture intérieure (12).

7. Dispositif d'implantation selon l'une quelconque des revendications précédentes, présentant exactement deux des dispositifs de transport (2a, 2b) disposés en vis-à-vis par rapport au tube de canule (15).

8. Dispositif d'implantation selon l'une quelconque des revendications précédentes, dans lequel une longueur des broches (3a, 3b) est respectivement égale ou supérieure à 100 mm.

9. Dispositif d'implantation selon l'une quelconque des revendications précédentes, dans lequel un pas des broches (3a, 3b) s'élève à 6 mm.

10. Dispositif d'implantation selon l'une quelconque des revendications précédentes, dans lequel la broche (3a, 3b) est conçue comme un filetage trapézoïdal.

11. Dispositif d'implantation selon l'une des revendications 2 à 11, dans lequel le boîtier (1) présente une échelle linéaire (10) sur une face extérieure, et dans lequel l'adaptateur (6) présente un élément (8) qui dépasse à travers le boîtier (1) par une encoche (9) s'étendant parallèlement à l'échelle (10) à côté de celle-ci.
